(19) Europäisches Patentamt
European Patent Office
Office européen des brevets

(11) **EP 2 015 056 B1**

(12) **EUROPÄISCHE PATENTSCHRIFT**

(45) Veröffentlichungstag und Bekanntmachung des Hinweises auf die Patenterteilung:
**07.04.2010   Patentblatt 2010/14**

(51) Int Cl.:
***G01N 33/22*** *(2006.01)*

(21) Anmeldenummer: **07013348.3**

(22) Anmeldetag: **07.07.2007**

(54) **Verfahren und Sensor zur Bestimmung einer brenntechnisch relevanten Größe eines Gasgemisches**

Method and sensor for determining a significant value in combustibility terms of a gas mixture

Procédé et capteur destinés à la détermination d'une taille pertinente pour la combustion d'un mélange de gaz

(84) Benannte Vertragsstaaten:
**AT BE BG CH CY CZ DE DK EE ES FI FR GB GR HU IE IS IT LI LT LU LV MC MT NL PL PT RO SE SI SK TR**

(43) Veröffentlichungstag der Anmeldung:
**14.01.2009   Patentblatt 2009/03**

(73) Patentinhaber: **MEMS AG**
**5405 Baden-Dättwil (CH)**

(72) Erfinder:
• **Pretre, Philippe**
**5405 Baden-Dättwil (CH)**
• **Matter, Daniel**
**5200 Brugg (CH)**
• **Suter, Tobias**
**8802 Kilchberg (CH)**
• **Kempe, Andreas**
**8049 Zürich (CH)**

(56) Entgegenhaltungen:
**EP-A- 0 484 645       EP-A- 1 193 488**
**EP-A- 1 265 068       WO-A-2004/008136**
**DE-A1- 4 118 781       DE-A1- 19 908 664**

**Beschreibung**

Die Erfindung bezieht sich auf ein Verfahren und auf einen Sensor zur Bestimmung einer brenntechnisch relevanten Größe eines Gasgemisches, insbesondere Erdgasgemisches. Unter einer brenntechnisch relevanten Größe wird insbesondere der Energieinhalt des Gasgemisches, ausgedrückt durch den Brennwert oder den Wobbe-Index verstanden. Unter der brenntechnisch relevanten Größe kann z. B. auch die Methanzahl verstanden werden.

Die Erfindung kann nebst der Steuerung von Verbrennungsprozessen (Gasfeuerungsregelung) auch zur Motorregelung von (Erd)gasfahrzeugen verwendet werden.

Aus der DE 41 18 781 A1 sind ein Verfahren und eine Vorrichtung zur verbrennungslosen Bestimmung der Wobbezahl und/oder des Brennwertes eines Gases bekannt, wobei der Volumenstrom gemessen und weitere charakteristische Kenngrößen des Gases, wie Druckabfall, Dichte, Viskosität gemessen und/oder konstant gehalten werden. Um eine hohe Messgenauigkeit zu erreichen, ist vorgesehen, dass ein Massenstrom thermisch gemessen wird und dass aus dem Volumen- und dem Massenstrom sowie mindestens einer weiteren der genannten Kenngrößen mit Hilfe von Näherungsfunktionen die Wobbezahl und/oder der Brennwert bestimmt und angezeigt oder weiterverarbeitet werden.

Aus der DE 101 22 039 A1 sind ein Verfahren und eine Vorrichtung zum Bestimmen eines für die Beschaffenheit eines Gases repräsentativen Kennwertes bekannt, wobei das Gas mit konstantem Volumenstrom durch eine Messleitung geführt und dort von einer Heizung mit konstanter Leistung beheizt wird. Zwei Temperatursensoren erfassen die Gastemperatur stromauf und stromab der Heizung. Die daraus abgeleitete Temperaturdifferenz ist proportional der spezifischen Wärme des Gases und damit zur Gasbeschaffenheit. Schwankungen der Temperatur des durch die Messleitung geführten Gasstromes werden kompensiert.

Aus der EP 1 265 068 A1 sind ein Verfahren und eine Vorrichtung zur Bestimmung einer Änderung des Heizwertes eines Gasgemisches bekannt, wobei die Diffusivität innerhalb des Gasgemisches bei einem konstanten, vordefinierten Massenstrom mittels eines Heizwertsensors gemessen wird. Der Heizwertsensor weist zwei in Flussrichtung nacheinander angeordnete Temperatursensorelemente, ein dazwischen angeordnetes Heizelement und Messmittel zur Ermittlung der Diffusivität aus Signalen der Temperatursensoren auf. Die gemessene Diffusivität wird mit dem Referenzwert eines Kalibriergases mit bekanntem Heizwert verglichen. Dabei ist eine Abweichung der gemessenen Diffusivität von dem Referenzwert ein Maß für die Änderung des Heizwertes des Gasgemisches. Dies ermöglicht eine einfache und kostengünstige Bestimmung der Änderungen von Zusammensetzungen von Gasgemischen und somit von relativen Heizwertänderungen. Als Mittel zur Erzeugung eines konstanten Massenflusses wird eine sonische (kritische) Düse eingesetzt.

Aus der DE 199 08 664 A1 ist ein Gaszähler mit einem Anemometer zur Bestimmung einer konsumierten Gasmenge bekannt, welches mittels Messung einer Temperaturänderung eines das Anemometer durch- oder überströmenden Gases die Gasmassendurchflussrate bestimmt. Das Anemometer kann als CMOS-Anemometer (Complementary Metal Oxide Semiconductor) ausgebildet und in einem Bypassrohr zu einem Hauptleitungsrohr einer Gasleitung angeordnet sein. Der Gaszähler weist ferner als Messkorrekturmittel für das Anemometer einen Dichtesensor zur Bestimmung der Dichte eines strömenden Gases auf. Dichtesensor und Anemometer können in einem gemeinsamen Chip integriert sein.

Aus der WO 2004/008136 A1 ist ein Verfahren zur Bestimmung der Gasbeschaffenheit von Brenngasen bekannt, bei dem mittels Korrelationsverfahren aus messtechnisch an dem Brenngas ermittelten Messgrößen Gaskenngrößen ermittelt werden, die Aussagen über die Gasbeschaffenheit des Brenngases erlauben, wobei als Messgröße die Wärmeleitfähigkeit $\lambda$, die Wärmekapazität $c_p$ und der Kohlenstoffdioxidanteil $XCO_2$ des jeweiligen Brenngases gemessen und daraus unter Einbeziehung bekannter Stoffgrößen für typische Brenngase die Gaskenngrößen berechnet werden, wie z. B. Brennwert, Dichte, Wobbeindex, Methanzahl etc.

Die traditionell am häufigsten verwendete Methode zur Bestimmung einer Gasgemisch-zusammensetzung bedient sich eines Gaschromatographen. Sie hat off-line Charakter, da nebst dem Messgas verschiedene andere Komponenten zur Analyse benötigt werden. Die erhältlichen Geräte sind zudem keine Massenmarktprodukte und wegen großen Temperaturschwankungen, Empfindlichkeit gegenüber mechanischen Erschütterungen usw. kaum für den Einsatz unter erschwerten Bedingungen tauglich.

Wichtiger als die Gasgemisch-Zusammensetzung sind aber oft physikalische Eigenschaften der Gasmischung wie etwa Energieinhalt, Brennwert oder Wobbe-Index bei Erdgasanwendungen wie Gasfeuerungsregelungen oder Motorregelungen bei Erdgasfahrzeugen (NGV: Natural Gas Vehicle).

Das aus der DE 101 22 039 A1 bekannte Verfahren bzw. die Vorrichtung betreffen einen "Gas Property Identifier" GPI. Dieses Gerät basiert auf einem modifizierten, thermischen Massendurchflussmesser. Die Modifikationen bestehen in erster Linie in einer aufwändigen Druck- und Differenzdruckregelung zur möglichst genauen Konstanthaltung des Messvolumenstroms. Die Ableitung des Brennwerts erfolgt über eine (lineare) Korrelation zum gemessenen Temperaturdifferenzsignal $\Delta T$ zweier bezüglich einer Heizwicklung stromauf- bzw. stromabwärts liegender Temperatursensoren $\Delta T=T2-T1$. Dabei ist T1 die Gastemperatur stromaufwärts und T2 die Gastemperatur stromabwärts. Nachteil ist der hohe Herstellungspreis, was Massenmarktanwendungen ausschließt. Der Erfindung liegt die Aufgabe zugrunde, ein Verfahren zur Bestimmung einer brenntechnisch relevanten Größe eines Gasgemisches anzugeben, womit die Herstellung eines kostengünstigen und präzisen Sensors ermöglicht wird. Des Weiteren soll ein nach dem erfindungsgemäßen Verfahren arbeitender Sensor angegeben werden.

Diese Aufgabe wird bezüglich des Verfahrens erfindungsgemäß gelöst durch ein Verfahren zur Bestimmung einer brenntechnisch relevanten Größe eines Gasgemisches, insbesondere des Energieinhalts des Gasgemisches, ausgedrückt durch den Brennwert oder den Wobbe-Index, mittels einer Korrelation zwischen der brenntechnisch relevanten Größe zu dem Verhältnis $c_p/\lambda$ aus der Wärmekapazität $c_p$ und der Wärmeleitfähigkeit $\lambda$ dieses Gasgemisches,

- wobei das Verhältnis $c_p/\lambda$ mit einem integrierten CMOS-Hitzedrahtanemometer bestimmt wird, während das CMOS-Hitzedrahtanemometer mit einem konstanten Massenfluss beaufschlagt wird,
- wobei der konstante Massenfluss mit einer kritischen Düse erzeugt wird,
- wobei die Wärmeleitfähigkeit $\lambda$ des Gasgemisches mit dem integrierten CMOS-Hitzedrahtanemometer separat bestimmt wird,
- und wobei die Korrelation eines zu erwartenden Massenflussfehlers zur Wärmeleitfähigkeit $\lambda$ des Gasgemisches herangezogen wird, um den Massenflussfehler zu erhalten oder zu korrigieren.

Diese Aufgabe wird bezüglich des Sensors gemäß einer ersten Variante erfindungsgemäß gelöst durch einen Sensor mit einem Sensorblock mit CMOS-Hitzedrahtanemometer, welcher an seinem Gas-Einlass mit dem Gas-Auslass einer kritischen Düse und an seinem Gas-Auslass mit einem Absperrventil verbunden ist, wobei an den Gas-Einlass der kritischen Düse eine zu einer Gashauptleitung führende Messleitung angeschlossen ist und wobei am Gas-Auslass des Absperrventils ein Aussendruck ansteht, der kleiner als die Hälfte des kritischen Drucks der kritischen Düse ist.

Diese Aufgabe wird bezüglich des Sensors gemäß einer zweiten Variante erfindungsgemäß gelöst durch einen Sensor mit einem Sensorblock mit CMOS-Hitzedrahtanemometer, welcher an seinem Gas-Einlass über ein Absperrventil mit dem Gas-Auslass einer kritischen Düse verbunden ist, wobei an den Gas-Einlass der kritischen Düse eine zu einer Gashauptleitung führende Messleitung angeschlossen ist und wobei am Gas-Auslass des Sensorblocks ein Aussendruck ansteht, der kleiner als die Hälfte des kritischen Drucks der kritischen Düse ist.

Die mit der Erfindung erzielbaren Vorteile bestehen insbesondere darin, dass aufgrund der kostengünstigen Herstellbarkeit des nach dem erfindungsgemäßen Verfahren arbeitenden Sensors mit CMOS-Hitzdrahtanemometer und kritischer Düse Massenmarktanwendungen möglich sind. Es ist eine hohe Messgenauigkeit im Bereich $\pm$ 1% erzielbar.

Weitere Vorteile sind aus der nachstehenden Beschreibung ersichtlich.

Vorteilhafte Ausgestaltungen der Erfindung sind in den Unteransprüchen gekennzeichnet.

Die Erfindung wird nachstehend anhand der in der Zeichnung dargestellten Ausführungsbeispiele erläutert. Es zeigen:

Fig. 1, 2a, 2b  drei Ausführungsbeispiele für den schematischen Aufbau eines Sensors (Gasartenbestimmungssensor) zur Energieinhaltsbestimmung von brennbaren Gasen,

Fig. 3  eine angestrebte Korrelation zwischen dem Brennwert und dem Verhältnis $C_p/\lambda$ aus der Wärmekapazität $C_p$ und der Wärmeleitfähigkeit $\lambda$ verschiedener Erdgase,

Fig. 4  eine angestrebte Korrelation zwischen dem Wobbe-Index und dem Verhältnis $C_p/\lambda$ aus der Wärmekapazität $C_p$ und der Wärmeleitfähigkeit $\lambda$ verschiedener Erdgase,

Fig. 5  den Korrekturfaktor $C_d$ als Funktion der idealen Reynoldszahl $Re^*_{ideal}$ für verschiedene Gase,

Fig. 6  den Massenflussfehler einer mit Verbundgas geeichten kritischen Düse in Abhängigkeit von der Wärmeleitfähigkeit $\lambda$.

Die Idee der Erfindung ist die Energieinhaltbestimmung, z.B. Brennwert- oder Wobbe-Index, eines Erdgasgemisches basierend auf einer mikrothermischen Massenmessung bei konstantem Fluss, wobei die Konstanthaltung des Flussparameters (Massen- oder Volumenstrom) nicht über eine aufwändige Regelung sondern mittels einer kritischen Düse in Kombination mit den der mikrothermischen Messmethode inhärenten Korrekturmöglichkeiten bezüglich Durchflussfehler erfolgt. Ausgegangen wird dabei von der das System beschreibenden, eindimensionalen Wärmeleitungsgleichung für nicht kompressible Medien

$$\frac{c_p}{\lambda} \cdot \rho \, v_x \cdot \frac{d}{dx} T = \nabla^2 T + \frac{1}{\lambda} \Theta \,. \qquad\qquad (1)$$

wobei

$V_x$     die Komponente der mittleren Fliessgeschwindigkeit (Geschwindigkeitsvektor) $\bar{v}$ in x-Richtung, d. h. entlang des Gasstromes,

T     die Temperatur,

$C_p$     die Wärmekapazität des Gases bei konstantem Druck,

$\rho$     die Dichte,

$\lambda$     die Wärmeleitfähigkeit des Gases,

$\nabla^2 T$     Laplace-Operator, angewandt auf die Temperatur T, wobei

$$\nabla^2 = \left(\frac{d}{d_x}\right)^2 + \left(\frac{d}{dy}\right)^2 + \left(\frac{d}{dz}\right)^2$$

bedeuten. Da das Gas (Gasstrom) nur in x-Richtung fließt, werden die Komponenten vy bzw. $V_z$ in y-Richtung bzw. in z-Richtung der mittleren Fliessgeschwindigkeit $\bar{v}$ zu Null angenommen. Auch beim Laplace-Operator $\nabla^2$ wird angenommen, dass dessen y-Komponente und z-Komponente vernachlässigbar sind. $\Theta$ mit der Einheit Watt/$m^3$ beschreibt den Quellterm des Heizelements. Der Quellterm ist im Prinzip der Heizdraht eines CMOS-Hitzdrahtanemometers, der Wärmeenergie in das System speist. Aus Gleichung (1) wird ersichtlich, dass der Massenfluss $\rho \cdot v_x$ und das Verhältnis $c_p/\lambda$ aus der Wärmekapazität $c_p$ und der Wärmeleitfähigkeit $\lambda$ komplementär eingehen, während die Wärmeleitfähigkeit $\lambda$ wegen des Quellterms $\Theta$ separat auf die Lösung der Gleichung (1) einwirkt. Können demnach der Massenfluss $p.v_x$ konstant gehalten und die Wärmeleitfähigkeit $\lambda$ des Gases separat gemessen werden, lässt sich auf das Verhältnis $C_p/\lambda$ schließen.

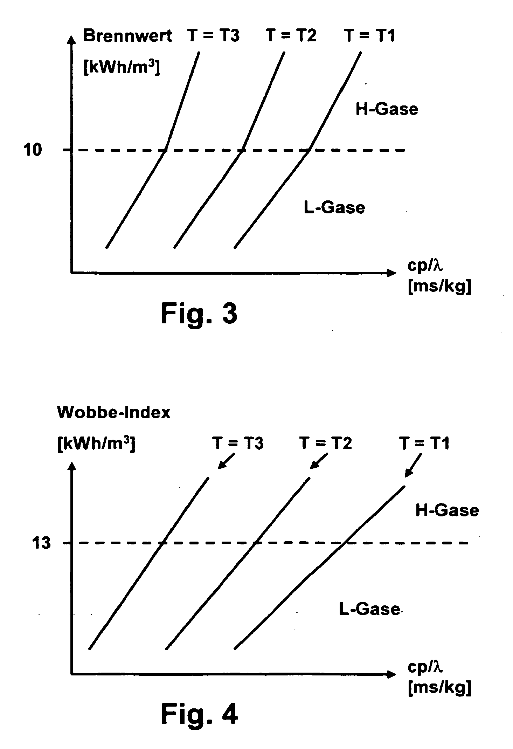

In Fig. 3 ist eine angestrebte Korrelation zwischen dem Brennwert (Ordinate, y-Achse, Einheit kWh/$m^3$) und dem Verhältnis $c_p/\lambda$ (Abszisse, x-Wert, Einheit ms/kg) aus der Wärmekapazität $c_p$ und der Wärmeleitfähigkeit $\lambda$ verschiedener Erdgase dargestellt. Es sind drei Kurven für drei unterschiedliche Temperaturen T = T1, T = T2, T = T3 für H-Gase (Brennwert über 10 kWh/$m^3$) und L-Gase (Brennwert unter ca. 10 kWh/$m^3$) zu erkennen.

In Fig. 4 ist eine angestrebte Korrelation zwischen dem Wobbe-Index (Ordinate, y-Achse, Einheit kWh/$m^3$) und dem Verhältnis $C_p/\lambda$ (Abszisse, x-Wert, Einheit ms/kg) aus der Wärmekapazität $C_p$ und der Wärmeleitfähigkeit $\lambda$ verschiedener Erdgase dargestellt. Es sind ebenfalls drei Kurven für drei unterschiedliche Temperaturen T = T1, T = T2, T = T3 für H-Gase (Wobbe-Index über ca. 13 kWh/$m^3$) und L-Gase (Wobbe-Index unter ca. 13 kWh/$m^3$) zu erkennen.

Wie Fig. 3 bzw. Fig. 4 zeigen, besteht zwischen diesem Verhältnis $C_p/\lambda$ und dem Brennwert (siehe Fig. 3) bzw. dem Wobbe-Index (siehe Fig. 4) eine hinreichend gute Korrelation. Dabei gilt für beide Figuren 3 und 4: T1 = 253 K, T2 = 293 K, T3 = 333 K.

Die Trennung in H-Gase (z. B. Erdgas aus Russland und der Nordsee) und L-Gase (z. B. Erdgas aus Holland) bei einem Brennwert von ca. 10 kWh/$m^3$ bzw. einem Wobbe-Index von ca. 13 kWh/$m^3$ ist definitionsgemäß (für eine Temperatur von 0˚C und einen Druck von 1013.25 mbar), die Separation entlang der $C_p/\lambda$-Achse hingegen entspricht der gesuchten Korrelation. Die eingezeichneten Geradenabschnitte sind nur als Gedankenhilfe zu verstehen. Im Prinzip kann jede eineindeutige Funktion von $C_p/\lambda$ verwendet werden.

Da mit dem Sensorelement inhärent die Temperaturen T1, T2 und T3 gemessen werden können, kann mit ersterem die Temperaturabhängigkeit kompensiert werden. Der Einfluss des Drucks auf die Messung des Sensorelements ist im relevanten Druckbereich völlig vernachlässigbar.

Die Voraussetzung eines konstanten Massenflusses lässt sich in der Praxis am kostengünstigsten mittels einer kritischen Düse verwirklichen. Zur kritischen Düse wird ausdrücklich auf Aaron N. Johnson, "Numerical characterization of the discharge coefficient in critical nozzles", Thesis, Pennsylvania State Universitiy, 2000, Seiten 1-2, 10-18, 64-65 hingewiesen.

In der Theorie des eindimensionalen, reibungsfreien Flusses errechnet sich der "ideale" Massenfluss $\dot{m}_{ideal}$ einer kritischen Düse zu

$$\dot{m}_{ideal} = \frac{Pi \cdot A \cdot C^{\bullet}}{\sqrt{Ti} \cdot \sqrt{R/MW}} \tag{2}$$

wobei

Pi   den Vordruck, d. h. den Druck vor der Düse in der Gashauptleitung,
Ti   die Vortemperatur, d. h. die Temperatur vor der Düse in der Gashauptleitung,
R   die Gaskonstante,
A   den Düsenquerschnitt,
MW   das Molekulargewicht,
C*   den kritischen Flussfaktor

bedeuten, wobei letzterer eine Funktion des isentropen Koeffizienten $y = C_p/C_v$ ist, wobei $c_v$ die Wärmekapazität des Gases bei konstantem Volumen ist. Für den kritischen Flussfaktor C* gilt:

$$C^{\bullet} = \sqrt{\gamma}\left(\frac{\gamma+1}{2}\right)^{\frac{\gamma+1}{2(1-\gamma)}}$$

Es wird hierzu auf Aaron N. Johnson, "Numerical characterization of the discharge coefficient in critical nozzles", Thesis, Pennsylvania State Universitiy, 2000, Seiten 1-2, 10-18, 64-65 hingewiesen. Für das kritische Druckverhältnis Pi/Pa zwischen Eingangsdruck Pi (Vordruck) und Ausgangsdruck Pa hinter der Düse gilt die Voraussetzung

$$\frac{Pi}{Pa} \geq \left(\frac{\gamma+1}{2}\right)^{\gamma/(\gamma-1)},$$

um die Düse als kritische Düse betreiben zu können. Dieses Verhältnis liegt bei den meisten Gasen knapp unter zwei, d. h. wenn der Vordruck Pi rund doppelt so groß oder größer als der Ausgangsdruck Pa ist, hat man kritische Verhältnisse bei kritischem Druck $p_{krit}$, d. h. das Gas strömt dann mit Schallgeschwindigkeit durch den engsten Querschnitt der Düse.
Für reale Verhältnisse wird ein Korrekturfaktor $C_d$, "Discharge Coefficient" genannt, eingeführt:

$$C_d \equiv \frac{\dot{m}_{real}}{\dot{m}_{ideal}}, \tag{3}$$

wobei

$\dot{m}_{real}$   dem gemessenen Massenfluss entspricht,
$C_d$   traditionell als Funktion der idealen Reynoldszahl $Re^*_{ideal}$ angegeben wird,

und es zeigt sich, dass für einen weiten Bereich für alle hier relevanten Gase die Beziehung

$$C_d = a - \frac{b}{\sqrt{Re^{\bullet}_{ideal}}} \tag{4}$$

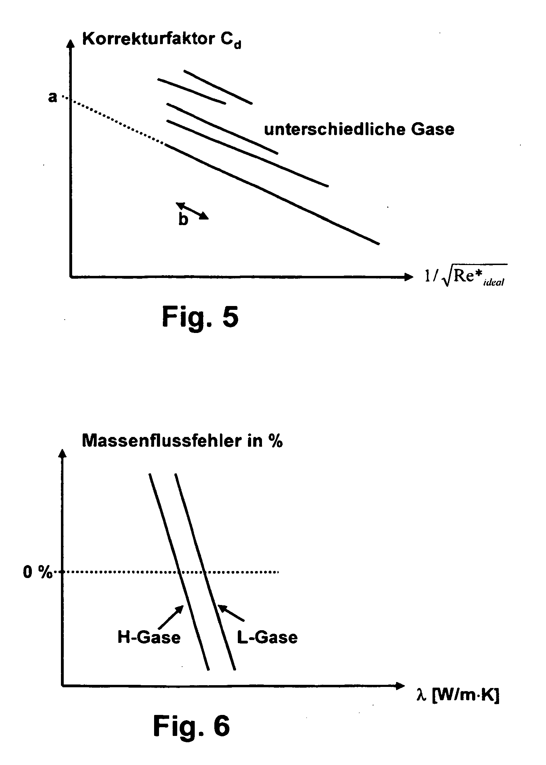

besteht, wie aus Fig. 5 hervorgeht, in welcher der Korrekturfaktor $C_d$ (Ordinate, y-Achse) als Funktion der idealen Reynoldszahl $Re^*_{ideal}$ (Abszisse, x-Achse) für verschiedene Gase dargestellt ist. Für Methan, Ethan, Stick- und Sauerstoff, die Hauptbestandteile aller hier betrachteten Erdgase, liest man sehr ähnliche Werte für a (Achsenabschnitt für x=0) und b (Steigung der Geraden) aus der Graphik ab, wobei Methan dominiert und für alle folgenden Berechnungen verwendet wird. Für Stickstoff $N_2$ ergibt sich z. B. für a=0,997 und für b=-3,618 (negative Steigung). $CO_2$ unterscheidet sich stark von den anderen Gasen, liegt aber in nur kleiner Konzentration vor, sodass keine merklichen Abweichungen zu Methan zu erwarten sind. Ein detailliertes Diagramm zu Fig. 5 ist in Aaron N. Johnson, "Numerical characterization of the discharge coefficient in critical nozzles", Thesis, Pennsylvania State Universitiy, 2000, Seite 64, Figure 3.1. zu finden.

Die wirklich gemessene Reynoldszahl $Re_{real}$ steht zur idealen Reynoldszahl $Re^*_{ideal}$ im gleichen Verhältnis wie die entsprechenden Massenflüsse, da

$$\text{Re}_{real} = \frac{4 \cdot \dot{m}_{real}}{\pi \cdot d \cdot \eta}$$

$$(5)$$

wobei

d     dem Düsendurchmesser und
$\eta$     der Vorkammerviskosität des Gases

entsprechen. Diese vier Gleichungen (2), (3), (4), (5) erlauben eine implizite Form für den Massenfluss $\dot{m}_{real}$ so dass numerisch nach diesem aufgelöst werden kann.

Die vorangehenden Betrachtungen zeigen, dass eine gewisse Gasabhängigkeit im Massenfluss kritischer Düsen besteht. Die Abweichung vom bei der Kalibrierung mit einem bestimmten Gas erhaltenen Sollwert ist für eine Reihe von Gasen wie oben dargestellt berechnet und in Fig. 6 über der Wärmeleitfähigkeit $\lambda$ der Gase aufgetragen, wobei in Fig. 6 der Massenflussfehler (Ordinate, y-Achse, in %) einer mit Verbundgas geeichten kritischen Düse in Abhängigkeit von der Wärmeleitfähigkeit $\lambda$ (Abszisse, x-Achse, Einheit $\dfrac{W}{m \cdot K}$ ) dargestellt ist.

Wie ersichtlich, besteht eine gute Korrelation des zu erwartenden Massenflussfehlers (Mass flow error in %) zur thermischen Leitfähigkeit $\lambda$ der Gase, was bei separater Messung der thermischen Leitfähigkeit $\lambda$ eine Korrektur des Massenflussfehlers zulässt (Massenflussfehlerkorrektur). Würde ohne Korrektur bei den gängigsten Erdgasen ein Fehler bis zu $\pm$ 5% auftreten, kann er mit Korrektur in den Bereich um $\pm$ 1% gebracht werden. Ähnlich wie in Fig. 3 bzw. Fig. 4 ist diese Korrektur von der Temperatur der Gase abhängig, Änderungen im Druck spielen aber keine Rolle, da die thermische Leitfähigkeit $\lambda$ und die Vorkammerviskosität $\eta$ nur sehr schwach vom Druck abhängig sind.

Eine Unterscheidung zwischen L- und H-Gasen ist hier nicht direkt möglich, was bei gegebener thermischer Leitfähigkeit unter Umständen zu einer falschen Massenflussfehlerkorrektur führen-kann. Ist jedoch in einem ersten Schritt einmal ein Wert für den Brennwert / Wobbe-Index aus den Figuren 3/4 bestimmt, kann bereits eine Unterscheidung zwischen L-Gas und H-Gas getroffen und in einem zweiten Schritt die korrekte Massenflussfehlerkorrektur gemäß Fig. 6 angewandt werden.

Die unter Fig. 6 erläuterte Korrekturmöglichkeit des Massenflussfehlers einer kritischen Düse ist von entscheidender Bedeutung und ermöglicht es, kritische Düsen für die Erzeugung eines quasi konstanten Massenflusses einzusetzen, was wiederum den Sensoraufbau wesentlich vereinfacht und die Herstellung eines kostengünstigen Sensors ermöglicht.

Die mikrothermische Massenmessung basiert auf einem integrierten CMOS Hitzdrahtanemometer. Zu dieser Technologie wird auf D. Matter, B. Kramer, T. Kleiner, B. Sabbattini, T. Suter, "Mikroelektronischer Haushaltsgaszähler mit neuer Technologie," Technisches Messen 71, 3 (2004), S. 137-146 hingewiesen. In dieser Hinsicht ist der vorgeschlagene Sensor äquivalent zum "Gas Property Identifier" GPI gemäß DE 101 22 039 A1. Der zusätzliche Vorteil der Integriertheit gegenüber dem GPI lässt die Bestimmung der Wärmeleitfähigkeit $\lambda$ eines Gasgemisches zu. Wie oben erwähnt, ist die separate Messung der Wärmeleitfähigkeit $\lambda$ Vorraussetzung zur Bestimmung des Verhältnisses $C_p/\lambda$ einerseits und zur Massenflussfehlerkorrektur andererseits.

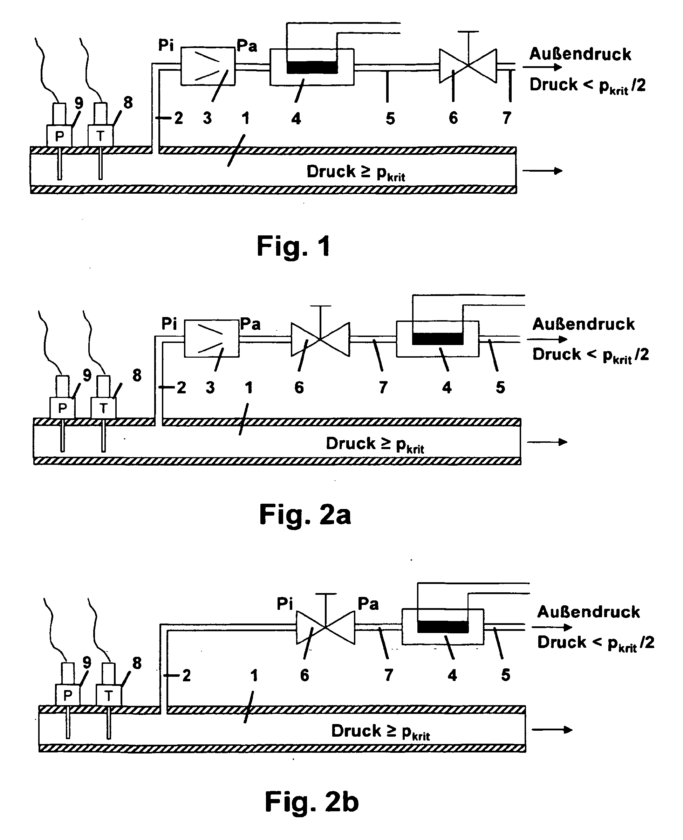

Ein einfacher Aufbau eines Sensors zur Energieinhaltsbestimmung von brennbaren Gasen ist in Fig. 1 gezeigt. Gas aus der Gashauptleitung 1, welche z.B. die Zufuhrleitung zum Motor des NGV ist, das unter einem Druck Pi größer als dem kritischen Druck $p_{krit}$ für eine kritische Düse 3 (sonische Düse) steht, wird über eine Messleitung 2 und die kritische Düse 3 zum mikrothermischen Sensor 4 (Sensorblock mit CMOS-Hitzdrahtanemometer) geführt. Ein Temperatur-Sensor 8 bzw. ein Druck-Sensor 9 dienen dienen zur Erfassung der Vortemperatur Ti bzw. des Vordrucks Pi - siehe hierzu auch Gleichung (2). Im Sensor 4 werden die Wärmeleitfähigkeit $\lambda$, das Verhältnis $C_p/\lambda$ sowie der Massen-

flussfehler bestimmt und über die Korrelation mit $C_p/\lambda$ die gesuchte Energieinhaltsgröße eruiert. Ein Absperrventil 6 am Auslass 5 des Sensors 4 dient dazu, den Gasfluss zu unterbrechen bzw. in Intervallen zur Messung zu öffnen und zwar gegen einen Aussendruck, der kleiner als typischerweise die Hälfte des kritischen Drucks $p_{krit}$ sein muss. Das am Auslass 7 des Absperrventils 6 ausfließende Gas kann in einem NGV direkt in das Ansaugsystem des Motors geleitet werden.

Beim Ausführungsbeispiel gemäß Fig. 2a ist im Unterschied zum Beispiel gemäß Fig. 1 das Absperrventil 6 zwischen die kritische Düse 3 und den Sensor 4 eingefügt, so dass am Auslass 5 des Sensors 4 ein Aussendruck ansteht, der kleiner als die Hälfte des kritischen Drucks $p_{krit}$ sein muss.

Bei Einsatz eines Magnetventils als Absperrventil 6 kann dieses prinzipiell gleichzeitig auch als kritische Düse 3 verwendet werden. Mit anderen Worten kann durch Einsatz des als Magnetventils ausgebildeten Absperrventils 6 die kritische Düse 3 entfallen. Fig. 2b zeigt das entsprechende Ausführungsbeispiel.

Bezugszeichenliste:

| | |
|---|---|
| 1 | Gashauptleitung |
| 2 | Messleitung |
| 3 | kritische (sonische) Düse |
| 4 | mikrothermischer Sensor (Sensorblock mit CMOS-Hitzdrahtanemometer) |
| 5 | Auslass am Sensorblock |
| 6 | Absperrventil |
| 7 | Auslass am Absperrventil |
| 8 | Temperatur-Sensor |
| 9 | Druck-Sensor |

| | |
|---|---|
| A | Düsenquerschnitt der kritischen Düse |
| a | Achsenabschnitt für x=0 (Fig. 5) |
| b | Steigung der Geraden (Fig. 5) |
| C* | kritischer Flussfaktor |
| $C_d$ | Korrekturfaktor, Discharge Coefficient |
| $C_p$ | Wärmekapazität des Gases bei konstantem Druck |
| $C_v$ | Wärmekapazität des Gases bei konstantem Volumen |
| d | Düsendurchmesser der kritischen Düse |
| $\dot{m}_{ideal}$ | idealer Massenfluss einer kritischen Düse |
| $\dot{m}_{real}$ | gemessener (realer) Massenfluss einer kritischen Düse |
| MW | Molekulargewicht des Gases |
| P | Druck eines Gases |
| Pa | Ausgangsdruck hinter der kritischen Düse |
| Pi | Vordruck der kritischen Düse, Eingangsdruck |
| $p_{krit}$ | kritischer Druck der kritischen Düse |
| R | Gaskonstante |
| $Re^*_{ideal}$ | ideale Reynoldszahl an der engsten Stelle der kritischen Düse |
| $Re_{real}$ | gemessene (reale) Reynoldszahl |
| T | Temperatur des Gases, Vortemperatur der kritischen Düse |
| $v_x$ | mittlere Fließgeschwindigkeit des Gases (Komponente in x-Richtung, d. h. entlang des Gasstromes) |
| $\eta$ | Vorkammerviskosität des Gases |
| y | isentroper Koeffizient |
| $\lambda$ | Wärmeleitfähigkeit des Gases |
| $\rho$ | Dichte des Gases |
| $\Theta$ | Quellterm des Heizelements |
| $\nabla$ | Laplace-Operator |

**Patentansprüche**

1. Verfahren zur Bestimmung einer brenntechnisch relevanten Größe eines Gasgemische, insbesondere des Energieinhalts des Gasgemisches, ausgedrückt durch den Brennwert oder den Wobbe-Index, mittels einer Korrelation zwischen der brenntechnisch relevanten Größe zu dem Verhältnis $c_p/\lambda$ aus der Wärmekapazität $c_p$ und der Wärmeleitfähigkeit $\lambda$ dieses Gasgemisches,

- wobei das Verhältnis $c_p/\lambda$ mit einem integrierten CMOS-Hitzedrahtanemometer bestimmt wird, während das CMOS-Hitzedrahtanemometer mit einem konstanten Massenfluss beaufschlagt wird,
- wobei der konstante Massenfluss mit einer kritischen Düse erzeugt wird,
- wobei die Wärmeleitfähigkeit $\lambda$ des Gasgemisches mit dem integrierten CMOS-Hitzedrahtanemometer separat bestimmt wird,
- und wobei die Korrelation eines zu erwartenden Massenflussfehlers zur Wärmeleitfähigkeit $\lambda$ des Gasgemisches herangezogen wird, um den Massenflussfehler zu erhalten oder zu korrigieren.

2. Verfahren nach Anspruch 1, **gekennzeichnet durch** ein Zwei-Schritt-Verfahren, wobei in einem ersten Schritt zunächst die brenntechnisch relevante Größe grob bestimmt wird, was eine Unterscheidung der Gasqualität in L-Gas und H-Gas ermöglicht und wobei in einem zweiten Schritt der zu erwartende Massenflussfehler in Kenntnis der Gasqualität erhalten und/oder korrigiert und die brenntechnisch relevante Größe fein bestimmt wird.

3. Sensor zur Durchführung eines Verfahrens mit den Merkmalen von Anspruch 1, mit einem Sensorblock (4) mit CMOS-Hitzedrahtanemometer, welcher an seinem Gas-Einlass mit dem Gas-Auslass einer kritischen Düse (3) und an seinem Gas-Auslass mit einem Absperrventil (6) verbunden ist, wobei an den Gas-Einlass der kritischen Düse (3) eine zu einer Gashauptleitung (1) führende Messleitung (2) angeschlossen ist und wobei am Gas-Auslass des Absperrventils (6) ein Aussendruck ansteht, der kleiner als die Hälfte des kritischen Drucks der kritischen Düse (3) ist.

4. Sensor zur Durchführung eines Verfahrens mit den Merkmalen von Anspruch 1, mit einem Sensorblock (4) mit CMOS-Hitzedrahtanemometer, welcher an seinem Gas-Einlass über ein Absperrventil (6) mit dem Gas-Auslass einer kritischen Düse (3) verbunden ist, wobei an den Gas-Einlass der kritischen Düse (3) eine zu einer Gashauptleitung (1) führende Messleitung (2) angeschlossen ist und wobei am Gas-Auslass des Sensorblocks (4) ein Aussendruck ansteht, der kleiner als die Hälfte des kritischen Drucks der kritischen Düse (3) ist.

5. Sensor nach Anspruch 4, **gekennzeichnet durch** den Einsatz eines Absperrventils (6), insbesondere eines Magnetventils, als kritische Düse (3).

**Claims**

1. Process for determining a combustion-relevant quantity of a gas mixture, notably the energy content of the gas mixture, expressed by the calorific value or Wobbe index, **characterized by** a correlation between the combustion-relevant quantity to the ratio $c_p/\lambda$ from the heat capacity $c_p$ and thermal conductivity $\lambda$ of the gas mixture

- whereas the ratio $c_p/\lambda$ is determined with an integrated CMOS-hot wire anemometer, while the integrated CMOS-hot wire anemometer is subjected to a constant mass flow,
- whereas the constant mass flow is produced with a sonic nozzle,
- whereas the thermal conductivity $\lambda$ of the gas mixture is determined separately with an integrated CMOS-hot wire anemometer,
- and whereas the correlation between an expected mass flow error and the thermal conductivity $\lambda$ of the gas mixture is used to determine an correct such mass flow error.

2. Process according to claim 1, **characterized by** a two-step procedure, whereas, in a first step, the combustion-relevant quantity is determined coarsely, allowing a discrimination of the gas quality into L-Gas and H-Gas, and whereas, in a second step, the knowledge of the gas quality allows for the determination and/or correction of the foreseeable mass flow error and for determining the combustion-relevant quantity finely.

3. Apparatus for the implementation of a process according to claim 1, **characterized by** a sensor block (4) with a CMOS-hot wire anemometer, whose gas inlet is connected to the gas outlet of a sonic nozzle (3) and whose gas outlet is connected to a stop valve (6), whereas the gas inlet of the sonic nozzle (3) is connected to a test line (2) leading to the main gas line (1) and whereas the gas outlet of the stop valve is subjected to an outside pressure smaller than half of the critical pressure of the sonic nozzle (3).

4. Apparatus for the implementation of a process according to claim 1, **characterized by** a sensor block (4) with a

EP 2 015 056 B1

CMOS-hot wire anemometer, whose gas inlet is connected via a stop valve (6) to the gas outlet of a sonic nozzle (3), whereas the gas inlet of the sonic nozzle (3) is connected to a test line (2) leading to the main gas line (1) and whereas the gas outlet of the sensor block is subjected to an outside pressure smaller than half of the critical pressure of the sonic nozzle (3).

**5.** Apparatus according to claim 4, **characterized by** means of a stop valve (6), notably a solenoid valve, as critical nozzle (3).


**Revendications**

**1.** Procédé pour déterminer une grandeur caractéristique de combustion d'un mélange gazeux, notamment le contenu énergétique du mélange gazeux, exprimé par le pouvoir calorifique ou l'indice de Wobbe, **caractérisé par** une corrélation entre la grandeur caractéristique de combustion et le rapport $c_p/\lambda$ de la chaleur massique $c_p$ et de la conductivité thermique $\lambda$ du mélange gazeux

- où le rapport $c_p/\lambda$ est déterminé avec un anémomètre à fil chaud intégré dans un circuit intégré CMOS, lorsque ledit circuit intégré est soumis à un débit massique constant,
- où le débit massique constant est produit avec une buse sonique,
- où la conductivité thermique $\lambda$ du mélange gazeux est déterminée séparément avec un anémomètre à fil chaud intégré dans un circuit intégré CMOS,
- et où la corrélation entre l'erreur prévisible du débit massique et la conductivité thermique $\lambda$ du mélange gazeux est utilisée pour déterminer et corriger une telle erreur de débit massique.

**2.** Procédé selon la revendication 1, **caractérisé par** une procédure en deux étapes, où lors de la première étape, la grandeur caractéristique de combustion est déterminée grossièrement, permettant la détermination du type du mélange gazeux, à savoir de type H ou L et où lors de la seconde étape, la connaissance du type du mélange gazeux permet de déterminer et / ou corriger l'erreur prévisible du débit massique et d'affiner la détermination de la grandeur caractéristique de combustion.

**3.** Dispositif pour la mise en oeuvre d'un procédé tel que défini dans la revendication 1, **caractérisé par** un bloc capteur (4) constitué d'un anémomètre à fil chaud intégré dans un circuit intégré CMOS, dont le conduit d'entrée du gaz est connecté à la sortie d'une buse sonique (3) et dont le conduit de sortie du gaz est connecté à une vanne d'arrêt (6), où le conduit d'entrée de la buse sonique (3) est connecté à une conduite de test (2) reliée à la conduite principale du gaz (1) et où le conduit de sortie de la vanne d'arrêt est soumis à une pression extérieure inférieure à la moitié de la pression critique de la buse sonique (3).

**4.** Dispositif pour la mise en oeuvre d'un procédé tel que défini dans la revendication 1, **caractérisé par** un bloc capteur (4) constitué d'un anémomètre à fil chaud intégré dans un circuit intégré CMOS, dont le conduit d'entrée du gaz est connecté à travers une vanne d'arrêt (6) à la sortie d'une buse sonique (3), où le conduit d'entrée du gaz de la buse sonique (3) est connecté à une conduite de test (2) reliée à la conduite principale du gaz (1) et où le conduit de sortie du gaz du bloc capteur est soumis à une pression extérieure inférieure à la moitié de la pression critique de la buse sonique (3).

**5.** Dispositif pour la mise en oeuvre d'un procédé tel que défini dans la revendication 4, **caractérisé par** une vanne d'arrêt (6), notamment une électrovanne, comme buse sonique (3).

9

**Fig. 1**

**Fig. 2a**

**Fig. 2b**

Fig. 3

Fig. 4

Korrekturfaktor $C_d$

a

unterschiedliche Gase

b

$1/\sqrt{Re^*_{ideal}}$

# Fig. 5

Massenflussfehler in %

0 %

H-Gase    L-Gase

$\lambda$ [W/m·K]

# Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 4118781 A1
- DE 10122039 A1
- EP 1265068 A1
- DE 19908664 A1
- WO 2004008136 A1

### In der Beschreibung aufgeführte Nicht-Patentliteratur

- **AARON N. JOHNSON.** Numerical characterization of the discharge coefficient in critical nozzles. Thesis, Pennsylvania State Universitiy, 2000, 1-210-1864-65
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146